# EUROPÄISCHE PATENTSCHRIFT

(11) **EP 0 802 176 B1**
(45) Veröffentlichungstag und Bekanntmachung des Hinweises auf die Patenterteilung: **19.07.2000**
(21) Anmeldenummer: 97105904.3
(22) Anmeldetag: 10.04.1997
(51) Int. Cl.: C07C 209/60

(54) **Verfahren zur Herstellung von Aminen aus Olefinen an mesoporösen Oxiden mit hoher Oberfläche**
Process for the preparation of amines from olefins in the presence of mesoporous oxides with high surface area
Procédé de préparation d'amines à partir d'oléfines en présence d'oxydes mésoporeux possédant une surface spécifique élevée

(30) Priorität: 19.04.1996 DE 19615482
(43) Veröffentlichungstag der Anmeldung: 22.10.1997
(73) Patentinhaber: BASF AKTIENGESELLSCHAFT, 67056 Ludwigshafen (DE)
(72) Erfinder: Eller, Karsten, Dr., 67059 Ludwigshafen (DE); Kummer, Rudolf, Dr., 67227 Frankenthal (DE); Müller, Ulrich, Dr., 67434 Neustadt (DE)

(56) Entgegenhaltungen:
- EP-A- 0 469 719
- EP-A- 0 670 286
- EP-A- 0 699 653
- EP-A- 0 752 409
- WO-A-91/11390
- PATENT ABSTRACTS OF JAPAN vol. 016, no. 298 (C-0958), 2.Juli 1992 & JP 04 082864 A (MITSUI TOATSU CHEM INC), 16.März 1992,

## Beschreibung

Die vorliegende Erfindung betrifft ein Verfahren zur Herstellung von Aminen durch Umsetzung von Ammoniak oder primären oder sekundären Aminen mit Olefinen bei erhöhten Temperaturen und Drükken in Gegenwart von mesoporösen Oxiden mit hoher Oberfläche.

Eine Übersicht über die Methoden zur Aminierung von Olefinen wird in "Functionalisation of Alkenes: Catalytic Amination of Monoolefins", J.J. Brunet et al., J. Mol. Catal., 49 (1989), Seiten 235 bis 259 gegeben.

Grundsätzlich gibt es zwei Katalysemechanismen. Das Olefin wird über einen Metallkomplex koordiniert. Diese aktivierte Spezies kann von dem nucleophilen Amin angegriffen werden und ein höher aminiertes Produkt bilden. Das Amin kann an Säurezentren oder an Metallzentren (via Metallamide) chemisorbiert werden und so aktiviert mit dem Olefin umgesetzt werden.

Gut geeignete Katalysatoren sind Zeolithe. Sie zeichnen sich durch eine hohe Anzahl an katalytisch aktiven Zentren aus, kombiniert mit einer großen Oberfläche. Die beschriebenen Zeolithe unterscheiden sich im Typ und in der Nachbehandlung (z.B. thermische Behandlung, Dealuminierung, Säurebehandlung, Metallioneneintausch, etc.). Beispiele hierfür finden sich in US-A-4 536 602, EP-A-101 921 oder DE-A-42 06 992.

Aus EP-A-133 938, EP-A-431 451 und EP-A-132 736 sind Verfahren bekannt, bei denen Bor-, Gallium-, Alumino- und Eisensilikatzeolithe für die Herstellung von Aminen aus Olefinen verwendet werden und auf die Möglichkeit der Dotierung dieser Zeolithe mit Alkali-, Erdalkali- und Übergangsmetallen hingewiesen wird.

Aus CA-A-2 092 964 ist ein Verfahren zur Herstellung von Aminen aus Olefinen bekannt, bei dem BETA-Zeolithe, die als kristalline Aluminosilikate bestimmter Zusammensetzung mit einer Porengröße von mehr als 5 Å definiert sind, eingesetzt werden. Bevorzugt werden metall- oder halogenmodifizierte Beta-Zeolithe eingesetzt.

EP-A-752 409 betrifft ein Verfahren zur Herstellung von Aminen durch Umsetzung von Gemischen, die man bei der Spaltung von Erdölfraktionen erhält, mit Ammoniak oder primären oder sekundären Aminen bei erhöhten Temperaturen und Drücken in Gegenwart eines Heterogenkatalysators.

EP-A-670 286 beschreibt mesoporöse Molekularsiebe, hergestellt aus anionischen Tensiden und Isopoly- oder Heteropolykationen von Metalloxiden der Gruppen IIIa, IIb, IIIb, IVb, Vb, VIb, VIIb des Periodensystems der Elemente, Be, Sn, Pb, Bi, Cu, Fe, Co, Ni, Ce, Th, U.

Ein besonderer Nachteil der Zeolithe als Katalysatoren liegt in ihrer aufwendigen Herstellung und damit in ihrem hohem Preis. Die selektive Synthese eines Molekularsiebs (z.B von Zeolithen) durch Hydrothermalsynthese erfordert die genaue Einhaltung vieler Parameter, wie etwa der Kristallisationszeit, der Kristallisationstemperatur, des Druckes oder der Alterungsschritte. Die bei einer Zeolithsynthese üblicherweise eingesetzte strukturdirigierende Verbindung (Templat) muß nach der Kristallisation entfernt werden. Die Entfernung des Templats geschieht in der Regel durch Calcinierung, wobei die organische Verbindung oxidativ abgebaut wird. Aus ökologischen und ökonomischen Gründen ist dies sehr negativ zu bewerten.

Die Herstellung von Kristallen bestimmter Größe oder Morphologie sowie die Präparation geträgerter Zeolithe, welche als Katalysatoren aufgrund verfahrenstechnischer Vorteile oft wünschenswert wäre, ist in der Regel nicht oder nur mit viel Aufwand möglich. Zeolithe besitzen eine sehr enge Porengrößenverteilung. Die Porengrößen variieren je nach Zeolithtyp von 4 bis ca. 12 Å.

Bei einer Zeolith-katalysierten Reaktion haben nur solche Moleküle Zugang zu den katalytisch aktiven Zentren im Inneren des Zeoliths, welche kleiner sind als die Porendimensionen. Reaktanden mit größeren Abmessungen sind vom Inneren der Poren ausgeschlossen.

Für die oben genannte Aminierungsreaktionen bedeutet dies, daß die katalytischen Zentren im Inneren des Zeolithen zur Herstellung von Aminen, welche größer sind als der Porendurchmesser, nicht zur Verfügung stehen.

Der vorliegenden Erfindung lag daher die Aufgabe zugrunde, den zuvor genannten Nachteilen abzuhelfen, insbesondere Katalysatoren für die Aminierung von Olefinen zu finden, deren Präparation deutlich einfacher ist als die der Zeolith-Katalysatoren und die eine günstige Porengrößenverteilung auch für voluminösere Aminmoleküle haben.

Der Einsatz von säuremodifizierten Montmorilloniten wird in EP-A-469 719 beschrieben. Die Verwendung von Fällkatalysatoren unter Kombination von zwei oder mehreren Metalloxiden ausgenommen der Kombination Si und Al wird in JP-04082864 dargestellt.

Aus der DE-A-44 31 093 sind oxidische Katalysatoren bekannt, die über den Sol-Gel-Prozess hergestellt werden. Die BET-Oberfläche der Gele liegt mit maximal 670 m²g⁻¹ jedoch deutlich unter der BET-Oberfläche der vorliegenden mesoporösen Oxide. Zudem ist die Bildung von Gelen ein metastabiler Vorgang und daher eine Maßstabsvergrößerung sehr schwierig.

Alle Verfahren zur Synthese von Aminen aus Olefinen an nichtzeolithischen Katalysatoren zeichnen sich durch eine geringe Amin-Ausbeute oder geringe Raum-Zeit-Ausbeute aus, oder führen zu einer raschen Desaktivierung der Katalysatoren.

Der vorliegenden Erfindung lag daher die Aufgabe zugrunde, diesen Nachteilen abzuhelfen.

Demgemäß wurde ein neues und verbessertes Verfahren zur Herstellung von Aminen der allgemeinen Formel I 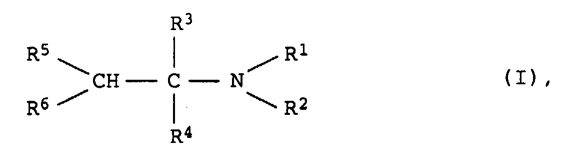 in der
- R¹,R²,R³,R⁴,R⁵,R⁶: Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
- R¹ und R²: gemeinsam eine gesättigte oder ungesättigte C₃- bis C₉-Alkylendikette und
- R³ oder R⁵: C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 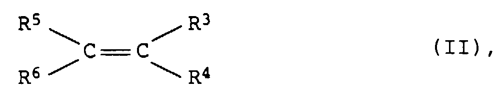 in der R³, R⁴, R⁵ und R⁶ die oben genannten Bedeutungen haben, mit Ammoniak oder primären oder sekundären Aminen der allgemeinen Formel III 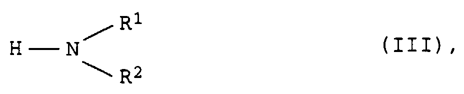 in der R¹ und R² die oben genannten Bedeutungen haben, bei Temperaturen von 200 bis 350°C und einem Druck von 100 bis 300 bar in Gegenwart eines Heterogenkatalysators gefunden, welches dadurch gekennzeichnet ist, daß man als Heterogenkatalysator ein mesoporöses Oxid mit SiO₂ als Hauptbestandteil und mit einer BET-Oberfläche in Pulverform von 400 bis 1400 m²/g, bei dem man röntgenographisch Reflexe im Winkelbereich von 2 bis 6° 2Theta beobachtet, einsetzt.

Das erfindungsgemäße Verfahren kann wie folgt durchgeführt werden:

Das Olefin II und Ammoniak oder das primäre oder sekundären Amin III kann bei Temperaturen von 200 bis 350°C, bevorzugt 220 bis 330°C, besonders bevorzugt 230 bis 320°C, und Drücken von 100 bis 300 bar, bevorzugt 120 bis 300 bar, besonders bevorzugt 140 bis 290 bar, in Gegenwart der mesoporösen Oxide als Katalysator z.B. in einem Druck-Reaktor umsetzt werden, und bevorzugt das erhaltene Amin abgetrennt und die nichtumgesetzten Einsatzstoffe zurückführt werden.

Das vorliegende Verfahren zeichnet sich durch eine sehr gute Ausbeute bei hoher Selektivität und bei hoher Raum-Zeit-Ausbeute aus. Zudem ist die Desaktivierung des Katalysators zurückgedrängt worden. Die Herstellung der Katalysatoren erfolgt in einfacher und gut reproduzierbarer Art und Weise.

Das erfindungsgemäße Verfahren zeichnet sich dadurch aus, daß bereits mit niedrigem Ammoniak- bzw. Amin- Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine III zusammen mit dem Olefin II im molaren Verhältnis von 1:1 bis 5:1 gemischt einem Festbettreaktor zuführt und bei einem Druck von 100 bis 300 bar und einer Temperatur von 200 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt.

Man kann einfach oder mehrfach ungesättigte Olefine II, insbesondere solche mit 2 bis 10 C-Atomen bzw. deren Mischungen und Polyolefine als Ausgangsstoffe verwenden. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden.

Die Lage des Gleichgewichts und damit der Umsatz zum gewünschten Amin ist sehr stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins und des Katalysators abhängig und liegt meist im Bereich von 200 bis 350°C.

Als Katalysatoren für die Aminierung von Olefinen eignen sich mesoporöse Oxide mit hoher Oberfläche und mit SiO₂ als Hauptbestandteil. Diese zeichnen sich dadurch aus, daß sie bei einer mittels Stickstoffadsorption (77 K) gemessenen Isotherme eine charakteristische Stufe im Relativdruck-Bereich von p/p° = 0,2 bis 0,4 aufweisen. Mittels transmissionselektronenmikroskopischer Analyse werden Mesoporen im Bereich von 2 bis 6 nm nachgewiesen und zudem röntgenographisch Reflexe im Winkelbereich 2 bis 6° 2Theta beobachtet. Neben Mesoporen können die erfindungsgemäßen Katalysatoren auch noch Mikroporen enthalten, die gemessene Porenverteilung, z.B. mittels der Stickstoffisotherme, ist dann bimodal. Die erzielbaren Oberflächen liegen bei den erfindungsgemäßen Katalysatoren in Pulverform bei 400 bis zu 1400 m²g⁻¹, bevorzugt bei 500 bis zu 1250 m²g⁻¹, besonders bevorzugt 700 bis zu 1250 m²g⁻¹ (gemäß BET-Auswertung), in verstrangter Form, je nach Binderanteil, bei 250 bis zu 900 m²g⁻¹, bevorzugt bei 300 bis zu 900 m²g⁻¹, besonders bevorzugt 350 bis zu 900 m²g⁻¹.

Hergestellt werden können die mesoporösen Oxide aus leicht löslichen Vorstufen der Oxide, z.B. durch saure oder alkalische Hydrolyse ihrer Metallsalze oder Alkoxide unter gleichzeitigem Zusatz von anionischen, kationischen oder nichtionischen Tensiden, etwa C₈- bis C₁₆-Trimethylammoniumsalze in Form der Chloride oder Bromide, oder C₈- bis C₁₆-Amine unter Zusatz von HCl (Gontier und Tuel; Zeolites 15 (1995), Seite 601 bis 610). Ebenso können als Template neutrale Amine (Mokaya und Jones, J. Chem. Soc., Chem. Commun. (1996), S. 981-982) oder Polyethylenoxide genommen werden (Bagshaw et al., Science 269 (1995), Seite 1242 bis 1244). Dabei bilden sich die mesoporösen Oxide innerhalb weniger Stunden bis Tage bei Temperaturen von Raumtemperatur bis 180°C. Nach dem Trocknen können die organischen Schablonenverbindungen durch einen Kalzinierschritt bei 350 bis 650°C unter Luft entfernt und die mesoporösen Oxide mit hohen Oberflächen erhalten werden.

Auch unter dem Namen MCM-41 oder M41S bekanntgewordenen mesoporösen Oxide mit hoher Oberfläche und regelmäßiger hexagonaler Anordnung der Poren zählen zu den erfindungsgemäßen Katalysatoren. Zu ihrer Herstellung kann auch wahlweise ein hydrothermaler Kristallisationsschritt durchgeführt werden.

Im erfindungsgemäßen Verfahren verwendete Heterogenkatalysatoren sind mesoporöse Oxide, z.B. nach der US-A-5 057 296, die aus einem oder mehreren Oxiden aus der Gruppe SiO₂, Al₂O₃, B₂O₃, Ga₂O₃, In₂O₃, TiO₂, ZrO₂, Nb₂O₅, Fe₂O₃, GeO₂, SnO₂, CeO₂ oder ThO₂, insbesondere aus der Gruppe SiO₂, Al₂O₃, B₂O₃ und TiO₂, zusammengesetzt sind, wobei SiO₂ Hauptbestandteil der mesoporösen Oxide oder besonders bevorzugt alleiniges Oxid ist.

Die erfindungsgemäßen mesoporösen Oxide mit hoher Oberfläche können als solche verformt werden, oder aber auch mit einem Bindemittel im Verhältnis 98:2 bis 40:60 Gew.-% zu Strängen oder Tabletten. Als Bindemittel eignen sich verschiedene Aluminiumoxide, bevorzugt Boehmit, amorphe Aluminosilikate mit einem SiO₂/Al₂O₃-Verhältnis von 25:75 bis 95:5, Siliciumdioxid, bevorzugt hochdisperses SiO₂, Gemische aus hochdispersem SiO₂ und hochdispersem Al₂O₃, hochdisperses TiO₂ sowie Tone. Nach der Verformung werden die Extrudate oder Preßlinge zweckmäßig bei 110°C/16 h getrocknet und bei 200 bis 500°C/2 bis 16 h calciniert, wobei die Calcinierung auch direkt (in situ) im Aminierungsreaktor erfolgen kann.

Zur Erhöhung der Selektivität, der Standzeit und der Anzahl der möglichen Regenerierungen kann man verschiedene Modifizierungen an den erfindungsgemäßen mesoporösen Oxiden mit hoher Oberfläche vornehmen.

Eine Modifizierung der Katalysatoren besteht darin, daß man die unverformten oder die verformten mesoporösen Oxide mit hoher Oberfläche mit Übergangsmetallen wie z.B. Ti, Zr, Mn, Fe, Mo, Cu, Zn, Cr, Edelmetallen und/oder seltenen Erdmetallen wie z.B. La, Ce oder Y ionenaustauschen bzw. dotieren kann.

Eine vorteilhafte Ausführungsform besteht darin, daß man die verformten erfindungsgemäßen mesoporösen Oxide mit hoher Oberfläche in einem Strömungsrohr vorlegt und bei 20 bis 100°C z.B. ein Halogenid, ein Acetat, ein Oxalat, ein Citrat oder ein Nitrat der oben beschriebenen Metalle in gelöster Form darüberleitet. Ein derartiger Ionenaustausch kann z.B. an der Wasserstoff-, Ammonium- und Alkaliform der erfindungsgemäßen mesoporösen Oxide mit hoher Oberfläche vorgenommen werden.

Eine weitere Möglichkeit der Metallaufbringung auf die erfindungsgemäßen mesoporösen Oxide mit hoher Oberfläche besteht darin, daß man das Material z.B. mit einem Halogenid, einem Nitrat, einem Acetat, einem Oxalat, einem Citrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten mesoporösen Oxiden mit hoher Oberfläche kann eine Nachbehandlung mit Wasserstoff und/oder mit Wasserdampf günstig sein.

Eine weitere Möglichkeit der Modifizierung besteht darin, daß man die erfindungsgemäßen mesoporösen Oxide mit hoher Oberfläche - verformt oder unverformt - einer Behandlung mit Säuren, wie Salzsäure (HCl), Flußsäure (HF), Schwefelsäure (H₂SO₄), Oxalsäure (HO₂C-CO₂H), Phosphorsäure (H₃PO₄) oder deren Gemischen unterwirft.

Eine besondere Ausführungsform besteht darin, daß man die erfindungsgemäßen mesoporösen Oxide mit hoher Oberfläche vor der Verformung mit einer der genannten Säuren 0,001 n bis 2 n, bevorzugt 0,05 bis 0,5 n, 1 bis 100 Stunden unter Rückfluß behandelt. Nach Abfiltrieren und Auswaschen wird in der Regel bei 100 bis 160°C getrocknet und bei 200 bis 600°C calciniert. Eine weitere besondere Ausführungsform liegt in einer Säure-Behandlung der erfindungsgemäßen mesoporösen Oxide mit hoher Oberfläche nach ihrer Verformung mit Bindemittel. Hierbei wird das erfindungsgemäße mesoporöse Oxid in der Regel 1 bis 3 Stunden zwischen 60 und 80°C mit einer 3 bis 25%igen, insbesondere mit einer 12 bis 20%igen Säure behandelt, anschließend ausgewaschen, bei 100 bis 160°C getrocknet und bei 200 bis 600°C calciniert. Auch hier kann die Calcinierung wieder direkt im Aminierungsreaktor erfolgen.

Eine andere Möglichkeit der Modifizierung ist gegeben durch einen Austausch mit Ammonsalzen, z.B. mit NH₄Cl oder mit Mono-, Di- oder Polyaminen. Hierbei wird das mit Bindemittel verformte mesoporöse Oxid in der Regel bei 60 bis 80°C mit 10 bis 25%iger, bevorzugt 20%iger NH₄Cl-Lösung 2 h kontinuierlich in gewichtsmäßiger Oxid/Ammonchlorid-Lösung von 1:15 ausgetauscht und danach bei 100 bis 120°C getrocknet.

Eine weitere Modifikation, die an den erfindungsgemäßen mesoporösen Oxiden mit hoher Oberfläche vorgenommen werden kann, ist eine Dealuminierung, bei der ein Teil der gegebenenfalls vorhandenen Aluminiumatome durch Silicium ersetzt wird oder die Oxide durch beispielsweise hydrothermale Behandlung in ihrem Aluminiumgehalt abgereichert werden. An eine hydrothermale Dealuminierung schließt sich vorteilhafterweise eine Extraktion mit Säuren oder Komplexbildnern an, um gebildetes Nichtgitteraluminium zu entfernen. Der Ersatz von Aluminium durch Silicium kann beispielsweise mit Hilfe von (NH₄)₂SiF₆ oder SiCl₄ erfolgen. Beispiele für Dealuminierungen finden sich in Corma et al., Stud. Surf. Sci. Catal. 37 (1987), Seiten 495 bis 503. In gleicher Weise können andere dreiwertige Elemente wie Bor, Eisen oder Gallium in ihrem Gehalt abgereichert werden.

Die Katalysatoren kann man als Stränge mit Durchmessern von z.B. 1 bis 4 mm als Kugeln oder als Tabletten mit z.B. 3 bis 5 mm Durchmesser für die Aminierung der Olefine einsetzen. Andere Formkörper sind ebenfalls möglich.

Aus dem beispielsweise zu Strängen verformten Katalysator kann man durch Mahlen und Sieben ein Wirbelgut in der Größe von 0,1 bis 0,8 mm erhalten.

Die Substituenten R¹, R², R³, R⁴, R⁵ und R⁶ in den Verbindungen I, II und III haben folgende Bedeutungen:
R¹,R²,R³,R⁴,R⁵,R⁶
- Wasserstoff,
- C₁- bis C₂₀-Alkyl, bevorzugt C₁- bis C₁₂-Alkyl, besonders bevorzugt C₁- bis C₈-Alkyl, wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
- C₂- bis C₂₀-Alkenyl, bevorzugt C₂- bis C₁₂-Alkenyl, besonders bevorzugt C₂- bis C₈-Alkenyl, wie Vinyl und Allyl,
- C₂- bis C₂₀-Alkinyl, bevorzugt C₂- bis C₈-Alkinyl, insbesondere C₂H und Propargyl,
- C₃- bis C₂₀-Cycloalkyl, bevorzugt C₃- bis C₁₂-Cycloalkyl, besonders bevorzugt C₅- bis C₈-Cycloalkyl, wie Cyclopentyl, Cyclohexyl, Cycloheptyl und Cyclooctyl,
- C₄- bis C₂₀-Alkyl-cycloalkyl, bevorzugt C₄- bis C₁₂-Alkyl-cycloalkyl, besonders bevorzugt C₅- bis C₁₀-Alkyl-cycloalkyl,
- C₄- bis C₂₀-Cycloalkyl-alkyl, bevorzugt C₄- bis C₁₂-Cycloalkyl-alkyl, besonders bevorzugt C₅- bis C₁₀-Cycloalkyl-alkyl,
- Aryl, wie Phenyl, 1-Naphthyl und 2-Naphthyl, bevorzugt Phenyl,
- C₇- bis C₂₀-Alkylaryl, bevorzugt C₇- bis C₁₆-Alkylaryl, bevorzugt C₇- bis C₁₂-Alkylphenyl, wie 2-Methylphenyl, 3-Methylphenyl, 4-Methylphenyl, 2-Ethylphenyl, 3-Ethylphenyl und 4-Ethylphenyl,
- C₇- bis C₂₀-Aralkyl, bevorzugt C₇- bis C₁₆-Aralkyl, bevorzugt C₇- bis C₁₂-Phenalkyl, wie Phenylmethyl, 1-Phenylethyl, 2-Phenylethyl,
R¹ und R²
- gemeinsam eine gesättigte oder ungesättigte C₃- bis C₉-Alkylendikette, bevorzugt -(CH₂)₄-, -(CH₂)₅-, -(CH₂)₇- und -CH=CH-CH=CH-,
R³ oder R⁵
- C₂₁- bis C₂₀₀-Alkyl, bevorzugt C₄₀- bis C₂₀₀-Alkyl, wie Polybutyl, Polyisobutyl, Polypropyl, Polyisopropyl und Polyethyl, besonders bevorzugt Polybutyl und Polyisobutyl,
- C₂₁- bis C₂₀₀-Alkenyl, bevorzugt C₄₀- bis C₂₀₀-Alkenyl, besonders bevorzugt C₇₀- bis C₁₇₀-Alkenyl,
R³ und R⁵
- gemeinsam eine C₂- bis C₁₂-Alkylendikette, bevorzugt eine C₃- bis C₈-Alkylendikette, besonders bevorzugt -(CH₂)₃-, - (CH₂)₄-,-(CH₂)₅-, -(CH₂)₆- und -(CH₂)₇-, insbesondere -(CH₂)₃- und -(CH₂)₄-.

### Beispiele

### Katalysatorsynthesen

### Katalysator A: Si-B-MPO

In einem 10 1 Kolben wurden 504 g Tetraethylorthosilikat (Si(OC₂H₅)₄), 3,24 g Borsäuretrimethylester (B(OCH₃)₃), 146,4 g Isopropanol und 720 g Ethanol vorgelegt und 30 min homogenisiert. Innerhalb von 30 min wurde langsam unter Rühren eine Mischung aus 120 g Dodecylamin, 1560 g dest. Wasser und 17,5 g 10 %-iger Salzsäure zugetropft. Die entstandene weiße Suspension wurde 20 h bei ca. 300 U/min gerührt, abfiltriert und mit dest. Wasser neutralgewaschen. Nach 24 h Trocknung bei 60°C wurde das Produkt bei 500°C für 10 h kalziniert. Das Pulver hatte eine BET-Oberfläche von 1084 m²g⁻¹.

60 g des Si-B-Oxids wurden mit 40 g Boehmit und 2 g Ameisensäure im Kneter kompaktiert und unter Wasserzusatz (124 ml) 45 min verknetet. In einer Strangpresse wurden mit einem Pressdruck von 40 bar 2 mm Stränge erzeugt und diese 4 h bei 120°C getrocknet und 16 h bei 500°C kalziniert.

Die fertigen Stränge hatten eine BET-Oberfäche von 794 m²g⁻¹ und eine deutlich bimodale Porenverteilung mit Maxima bei ca. 23 Å und ca. 60 Å.

### Katalysator B: Si-MPO

In einem 10 l Kolben wurden 505 g Tetraethylorthosilikat (Si(OC₂H₅)₄), 146,4 g Isopropanol und 720 g Ethanol vorgelegt und 30 min homogenisiert. Innerhalb von 30 min wurde langsam unter Rühren eine Mischung aus 120 g Dodecylamin, 1560 g dest. Wasser und 17,5 g 10 %-iger Salzsäure zugetropft. Die entstandene weiße Suspension wurde 20 h bei ca. 300 U/min gerührt, abfiltriert und mit dest. Wasser neutralgewaschen. Nach 24 h Trocknung bei 60°C wurde das Produkt bei 500°C für 5 h kalziniert. Das Pulver hatte eine BET-Oberfläche von 920 m²g⁻¹.

70 g des Si-Oxids wurden mit 46 g Boehmit und 2 g Ameisensäure im Kneter kompaktiert und unter Wasserzusatz (136 ml) 60 min verknetet. In einer Strangpresse wurden mit einem Pressdruck von 50 bar 2 mm Stränge erzeugt und diese 4 h bei 120°C getrocknet und 16 h bei 500°C kalziniert.

Die fertigen Stränge hatten eine BET-Oberfäche von 661 m²g⁻¹ und eine deutlich bimodale Porenverteilung mit Maxima bei ca. 25 Å und 80 bis 90 Å.

### Katalysator C: Ce/Si-MPO

40 g Katalysator B in Strangform wurden über Nacht bei 120°C getrocknet und anschließend mit einer Lösung aus 2,5 g Ce(NO₃)₃·6H₂O in 48 ml dest. Wasser versetzt. Nach einer halben Stunde Stehen lassen wurde das Wasser bei 80°C abgezogen, 2 h bei 120°C getrocknet und 2 h bei 540°C kalziniert.

Die fertigen Stränge enthielten 1,9 Gew.-% Cer.

### Katalysator D: Si-Al-MPO

In einem 10 l Kolben wurden 505 g Tetraethylorthosilikat (Si(OC₂H₅)₄), 6,34 g Aluminiumisopropylat (Al(O-isoC₃H₇)₃), 146,4 g Isopropanol und 720 g Ethanol vorgelegt und 30 min homogenisiert. Innerhalb von 30 min wurde langsam unter Rühren eine Mischung aus 120 g Dodecylamin, 1560 g dest. Wasser und 17,5 g 10 %-iger Salzsäure zugetropft. Die entstandene weiße Suspension wurde 20 h bei ca. 300 U/min gerührt, abfiltriert und mit dest. Wasser neutralgewaschen. Nach 24 h Trocknung bei 60°C wurde das Produkt bei 500°C für 5 h kalziniert. Das Pulver hatte eine BET-Oberfläche von 990 m²g⁻¹ und enthielt 43 Gew.-% Silicium sowie 0,53 % Aluminium.

70 g des Si-Al-Oxides wurden mit 46 g Boehmit und 2 g Ameisensäure versetzt, im Kneter kompaktiert und unter Wasserzusatz 120 min verknetet. In einer Strangpresse wurden mit einem Pressdruck von 40 bar 2 mm Stränge erzeugt und diese 16 h bei 110°C getrocknet und 16 h bei 500°C kalziniert.

Die fertigen Stränge hatten eine BET-Oberfäche von 641 m²g⁻¹ und eine deutlich bimodale Porenverteilung mit Maxima bei ca. 30 Å und 60 bis 200 Å.

### Katalysator E: SiO₂, niedrige Oberfläche (Vergleichsbeispiel)

Aus Wasserglas wurde durch Ansäuern mit Schwefelsäure SiO₂ ausgefällt. Nach Filtrieren wurde das Pulver sprühgetrocknet, zu 3 mm Strängen extrudiert und diese bei 650°C kalziniert. Die Oberfläche der kalzinierten Stränge betrug 173 m²g⁻¹.

### Katalysator F: MCM-41 (nach J. Am. Chem. Soc., 114 (1992), Seite 10834)

115,5 g Tetramethylammoniumbromid wurden zu 1500 ml Cetyltrimethylammoniumchlorid-Lösung [25 Gew.-% C₁₆H₃₃N(CH₃)₃Cl] gegeben, 30 Minuten gerührt und anschließend 272,6 g Natriumsilikat, 30 g Natriumaluminat, 187,5 g Aerosil® 200 und 1500 ml Wasser langsam zugegeben. Die Mischung wurde in einen Autoklaven überführt und bei Raumtemperatur 16 Stunden gerührt. Ohne Rühren wurde auf 160°C erhitzt und 3 Tage kristallisiert. Nach Abfiltrieren und Waschen wurde 12 Stunden bei 500°C kalziniert. Das Pulver besaß eine BET-Oberfläche von 640 m²g⁻¹, ein SiO₂/Al₂O₃-Verhältnis von 30:1 und Poren von ca. 43 Å.

100 g MCM-41 (zuvor hergestelltes Pulver) wurden mit 67 g Boehmit und 3 g Ameisensäure besetzt, im Kneter kompaktiert und unter Wasserzusatz (150 ml) 45 Minuten verknetet. In einer Strangpresse wurden mit einem Pressdruck von 80 bar Stränge von 2 mm erzeugt und diese 16 Stunden bei 110°C getrocknet und 16 Stunden bei 500°C kalziniert.

### Aminierungsbeispiele

Die Versuche wurden in einem Rohrreaktor (6 mm Innendurchmesser) unter isothermen Bedingungen bei 260 bis 300°C und einem Druck von 280 bar mit einem Gemisch aus Isobuten und Ammoniak im molaren Verhältnis von 1:1,5 durchgeführt. Die Reaktionsprodukte wurden im Gaschromatographen analysiert.

Die Ergebnisse sind in Tabelle 1 zusammengestellt und zeigen, daß die erfindungsgemäßen Katalysatoren deutlich höhere Ausbeuten als nach konventionellen Methoden hergestellte Katalysatoren auf Basis von Oxiden liefern und daß insbesondere die Aktivität durch Cer-Imprägnierung noch gesteigert werden kann.

**Tabelle 1**

| tert.-Butylamin (NH₃: C₄H₈ = 1,5) | | | | | | | |
|---|---|---|---|---|---|---|---|
| Katalysator | | Druck | Temperatur | tert.-Butylamin-Ausbeute [Gew.-%] | | | Litergewicht |
| Nr. | Al₂O₃ [Gew.-%] | [bar] | [°C] | WHSV 0,7 [g/g·h] | WHSV 1,5 [g/g·h] | WHSV 3 [g/g·h] | [kg/l] |
| A | 40 | 280 | 300 | 12,24 | 11,47 | 9,60 | 0,39 |
| B | 40 | 280 | 300 | 12,48 | 11,75 | 9,61 | 0,42 |
| C | 40 | 280 | 300 | 12,17 | 12,18 | 11,44 | 0,42 |
| D | 40 | 280 | 300 | 11,88 | 11,46 | 10,27 | 0,28 |
| E | | 280 | 300 | 2,01 | 0,92 | 0,52 | 0,37 |
| F | 40 | 280 | 300 | 12,86 | 11,20 | | 0,54 |

## Patentansprüche

1. Verfahren zur Herstellung von Aminen der allgemeinen Formel I 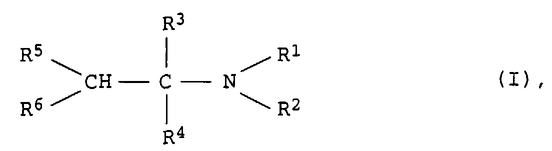 in der
R¹,R²,R³,R⁴,R⁵,R⁶ Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
R¹ und R² gemeinsam eine gesättigte oder ungesättigte C₃- bis C₉-Alkylendikette und
R³ oder R⁵ C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 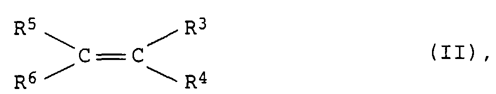 in der R³, R⁴, R⁵ und R⁶ die oben genannten Bedeutungen haben, mit Ammoniak oder primären oder sekundären Aminen der allgemeinen Formel III 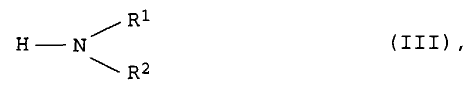 in der R¹ und R² die oben genannten Bedeutungen haben, bei Temperaturen von 200 bis 350°C und einem Druck von 100 bis 300 bar in Gegenwart eines Heterogenkatalysators, dadurch gekennzeichnet, daß man als Heterogenkatalysator ein mesoporöses Oxid mit SiO₂ als Hauptbestandteil und mit einer BET-Oberfläche in Pulverform von 400 bis 1400 m²/g, bei dem man röntgenographisch Reflexe im Winkelbereich von 2 bis 6° 2Theta beobachtet, einsetzt.

2. Verfahren zur Herstellung von Aminen I nach Anspruch 1, dadurch gekennzeichnet, daß man das gebildete Amin I abtrennt und die nichtumgesetzten Einsatzstoffe II und III zurückführt.

3. Verfahren zur Herstellung von Aminen nach den Ansprüchen 1 bis 2, dadurch gekennzeichnet, daß man als Olefin II Isobuten, Diisobuten, Cyclopenten, Cyclohexen oder Polyisobuten einsetzt.

4. Verfahren zur Herstellung von Aminen nach den Ansprüchen 1 bis 3, dadurch gekennzeichnet, daß man ein mesoporöses Oxid als Heterogenkatalysator einsetzt, das frei von Alkali- oder Erdalkali-Ionen ist.

5. Verfahren zur Herstellung von Aminen nach den Ansprüchen 1 bis 4, dadurch gekennzeichnet, daß man ein mesoporöses Oxid als Heterogenkatalysator einsetzt, das mit einem oder mehreren Übergangsmetallen dotiert ist.

6. Verfahren zur Herstellung von Aminen nach den Ansprüchen 1 bis 5, dadurch gekennzeichnet, daß man ein mesoporöses Oxid als Heterogenkatalysator einsetzt, das mit einem oder mehreren Elementen der Seltenen Erden dotiert ist.

7. Verfahren zur Herstellung von Aminen nach den Ansprüchen 1 bis 6, dadurch gekennzeichnet, daß man ein mesoporöses Oxid als Heterogenkatalysator einsetzt, das mit einer Säure, insbesondere einer aus der Gruppe Salzsäure, Flußsäure, Schwefelsäure, Phosphorsäure, Oxalsäure oder deren Gemischen, behandelt ist.

8. Verfahren zur Herstellung von Aminen nach den Ansprüchen 1 bis 7, dadurch gekennzeichnet, daß man einen ein mesoporöses Oxid enthaltenden Heterogenkatalysator einsetzt, der mit einem Bindemittel verformt und bei Temperaturen von 200 bis 600°C calciniert ist.

9. Verfahren zur Herstellung von Aminen nach den Ansprüchen 1 bis 8, dadurch gekennzeichnet, daß man ein mesoporöses Oxid mit einer Oberfläche von 500 bis 1250 m²g⁻¹ als Heterogenkatalysator einsetzt.

10. Verfahren zur Herstellung von Aminen nach den Ansprüchen 1 bis 9, dadurch gekennzeichnet, daß man als mesoporöses Oxid einen MCM-41 Typ einsetzt.

11. Verfahren zur Herstellung von Aminen nach den Ansprüchen 1 bis 10, dadurch gekennzeichnet, daß man als Heterogenkatalysator ein in Gegenwart eines kationischen oder nichtionischen Tensids oder neutralen Amins hergestelltes mesoporöses Oxid einsetzt.

## Claims

1. A process for preparing amines of the general formula I 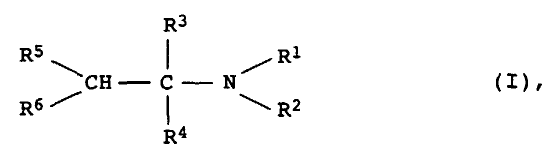 where
R¹,R²,R³,R⁴,R⁵,R⁶ are hydrogen, C₁-C₂₀-alkyl, C₂-C₂₀-alkenyl, C₂-C₂₀-alkynyl, C₃-C₂₀-cycloalkyl, C₄-C₂₀-alkyl-cycloalkyl, C₄-C₂₀-cycloalkyl-alkyl, aryl, C₇-C₂₀-alkylaryl or C₇-C₂₀-aralkyl,
R¹ and R² together form a saturated or unsaturated, divalent C₃-C₉-alkylene chain and
R³ and R⁵ are C₂₁-C₂₀₀-alkyl, C₂₁-C₂₀₀-alkenyl or together form a divalent C₂-C₁₂-alkylene chain,
by reacting olefins of the general formula II 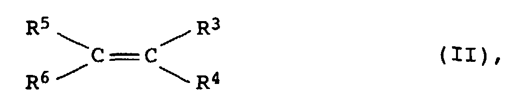 where R³, R⁴, R⁵ and R⁶ are as defined above, with ammonia or primary or secondary amines of the general formula III 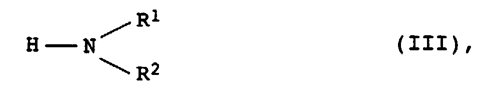 where R¹ and R² are as defined above, at from 200 to 350°C and a pressure of from 100 to 300 bar in the presence of a heterogeneous catalyst, wherein the heterogeneous catalyst used is a mesoporous oxide having SiO₂ as the main constituent and having a BET surface area in powder form of from 400 to 1400 m²/g, in which X-ray crystallographic reflections in the 2-theta range from 2 to 6° are observed.

2. A process for preparing amines I as claimed in claim 1, wherein the amine I formed is separated off and the unreacted starting materials II and III are recirculated.

3. A process for preparing amines as claimed in claim 1 or 2, wherein the olefin II used is isobutene, diisobutene, cyclopentene, cyclohexene or polyisobutene.

4. A process for preparing amines as claimed in any of claims 1 to 3, wherein the heterogeneous catalyst used is a mesoporous oxide which is free of alkali metal ions or alkaline earth metal ions.

5. A process for preparing amines as claimed in any of claims 1 to 4, wherein the heterogeneous catalyst used is a mesoporous oxide which is doped with one or more transition metals.

6. A process for preparing amines as claimed in any of claims 1 to 5, wherein the heterogeneous catalyst used is a mesoporous oxide which is doped with one or more elements of the rare earths.

7. A process for preparing amines as claimed in any of claims 1 to 6, wherein the heterogeneous catalyst used is a mesoporous oxide which has been treated with an acid, in particular an acid selected from the group consisting of hydrochloric acid, hydrofluoric acid, sulfuric acid, phosphoric acid, oxalic acid or mixtures thereof.

8. A process for preparing amines as claimed in any of claims 1 to 7, wherein a heterogeneous catalyst comprising a mesoporous oxide is shaped using a binder and calcined at from 200 to 600°C.

9. A process for preparing amines as claimed in any of claims 1 to 8, wherein the heterogeneous catalyst used is a mesoporous oxide having a surface area of from 500 to 1250 m²g⁻¹.

10. A process for preparing amines as claimed in any of claims 1 to 9, wherein the mesoporous oxide is of the MCM-41 type.

11. A process for preparing amines as claimed in any of claims 1 to 10, wherein the heterogeneous catalyst used is a mesoporous oxide prepared in the presence of a cationic or nonionic surfactant or neutral amine.

## Revendications

1. Procédé de préparation d'amines de formule générale I 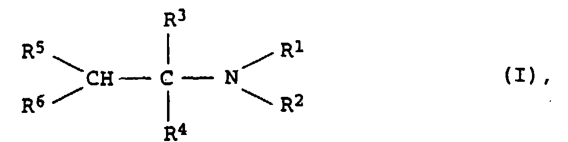 dans laquelle
R¹, R², R³, R⁴, R⁵, R⁶ représentent des atomes d'hydrogène, des groupements alkyle en C₁-C₂₀, alcényle en C₂-C₂₀, alcynyle en C₂-C₂₀, cycloalkyle en C₃-C₂₀, (alkyle en C₄-C₂₀)cycloalkyle, (cycloalkyle en C₄-C₂₀)alkyle, aryle, alkylaryle en C₇-C₂₀ ou aralkyle en C₇-C₂₀,
R¹ et R² représentent ensemble une dichaîne alkylène en C₃-C₉ saturée ou insaturée et
R³ ou R⁵ représentent un groupement alkyle en C₂₁-C₂₀₀, alcényle en C₂₁-C₂₀₀, ou bien ensemble une dichaîne alkylène en C₂-C₁₂,
par réaction d'oléfines de formule générale II 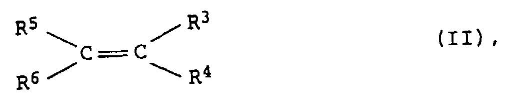 dans laquelle R³, R⁴, R⁵ et R⁶ prennent les significations susmentionnées, avec de l'ammoniac ou des amines primaires ou secondaires de formule générale III 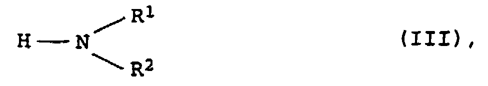 dans laquelle R¹ et R² prennent les significations susmentionnées, à des températures de 200 à 350°C et sous une pression de 100 à 300 bar en présence d'un catalyseur hétérogène, caractérisé en ce que l'on utilise en tant que catalyseur hétérogène un oxyde mésoporeux ayant SiO₂ comme constituant principal et ayant, sous forme de poudre, une surface BET de 400 à 1400 m²/g, pour lequel on observe des réflexions de rayons X dans un domaine angulaire 2 téta de 2 à 6°.

2. Procédé de préparation d'amines I selon la revendication 1, caractérisé en ce que l'on sépare l'amine I formée et qu'on recycle les matières de départ II et III n'ayant pas réagi.

3. Procédé de préparation d'amines selon la revendication 1 ou 2, caractérisé en ce que l'on utilise en tant qu'oléfine II de l'isobutène, du diisobutène, du cyclopentène, du cyclohexène ou du polyisobutène.

4. Procédé de préparation d'amines selon l'une quelconque des revendications 1 à 3, caractérisé en ce que l'on utilise en tant que catalyseur hétérogène un oxyde mésoporeux exempt d'ions alcalins ou alcalino-terreux.

5. Procédé de préparation d'amines selon l'une quelconque des revendications 1 à 4, caractérisé en ce que l'on utilise en tant que catalyseur hétérogène un oxyde mésoporeux dopé avec un ou plusieurs métaux de transition.

6. Procédé de préparation d'amines selon l'une quelconque des revendications 1 à 5, caractérisé en ce que l'on utilise en tant que catalyseur hétérogène un oxyde mésoporeux dopé avec un ou plusieurs éléments des terres rares.

7. Procédé de préparation d'amines selon l'une quelconque des revendications 1 à 6, caractérisé en ce que l'on utilise en tant que catalyseur hétérogène un oxyde mésoporeux traité avec un acide, en particulier un acide du groupe de l'acide chlorhydrique, l'acide fluorhydrique, l'acide sulfurique, l'acide phosphorique, l'acide oxalique ou un de leurs mélanges.

8. Procédé de préparation d'amines selon l'une quelconque des revendications 1 à 7, caractérisé en ce que l'on utilise un catalyseur hétérogène contenant un oxyde mésoporeux, qui a été façonné avec un liant et calciné à des températures de 200 à 600°C.

9. Procédé de préparation d'amines selon l'une quelconque des revendications 1 à 8, caractérisé en ce que l'on utilise en tant que catalyseur hétérogène un oxyde mésoporeux ayant une surface de 500 à 1250 m²g⁻¹.

10. Procédé de préparation d'amines selon l'une quelconque des revendications 1 à 9, caractérisé en ce que l'on utilise en tant qu'oxyde mésoporeux un type MCM-41.

11. Procédé de préparation d'amines selon l'une quelconque des revendications 1 à 10, caractérisé en ce que l'on utilise en tant que catalyseur hétérogène un oxyde mésoporeux préparé en présence d'un agent tensio-actif cationique ou non-ionique ou d'une amine neutre.
